# EUROPEAN PATENT SPECIFICATION

(11) **EP 3 690 435 B1**
(45) Date of publication and mention of the grant of the patent: **17.12.2025**
(21) Application number: 18860064.7
(22) Date of filing: 25.09.2018
(51) Int. Cl.: G01N 33/70, G01N 30/60, G01N 30/02

(54) **MARKER FOR HEALTH ASSESSMENT, AND APPLICATION THEREOF**
MARKER ZUR GESUNDHEITSBEURTEILUNG UND ANWENDUNG DAVON
MARQUEUR D'ÉVALUATION DE SANTÉ ET APPLICATION DE CE DERNIER

(30) Priority: 28.09.2017 CN 201710902571
(43) Date of publication of application: 05.08.2020
(73) Proprietor: Cai, Jianping, Beijing 100730 (CN)
(72) Inventor: CHEN, Zhe, Beijing 100730 (CN); CAI, Jianping, Beijing 100730 (CN)
(74) Representative: Gulde & Partner
(86) International application number: PCT/CN2018/107287
(87) International publication number: WO 2019/062702

(56) References cited:
- CN-A- 103 048 392
- ANDREOLI R ET AL: "Reference ranges of urinary biomarkers of oxidized guanine in (2'-deoxy)ribonucleotides and nucleic acids", FREE RADICAL BIOLOGY & MEDICINE, ELSEVIER INC, US, vol. 50, no. 2, 15 January 2011 (2011-01-15), pages 254 - 261, XP027593661, ISSN: 0891-5849, [retrieved on 20110107], DOI: 10.1016/J.FREERADBIOMED.2010.11.009
- TRANFO G ET AL: "Association of exposure to benzene and smoking with oxidative damage to nucleic acids by means of biological monitoring of general population volunteers", ENVIRONMENTAL SCIENCE AND POLLUTION RESEARCH, ECOMED, LANDSBERG, DE, vol. 24, no. 16, 14 March 2016 (2016-03-14), pages 13885 - 13894, XP036250715, ISSN: 0944-1344, [retrieved on 20160314], DOI: 10.1007/S11356-016-6366-1
- GAN WEI ET AL: "Age-dependent increases in the oxidative damage of DNA, RNA, and their metabolites in normal and senescence-accelerated mice analyzed by LC-MS/MS: Urinary 8-oxoguanosine as a novel biomarker of aging", FREE RADICAL BIOLOGY & MEDICINE,, vol. 52, no. 9, 1 May 2012 (2012-05-01), pages 1700 - 1707URL, XP002682476, ISSN: 0891-5849
- MANINI P ET AL: "Occupational exposure to low levels of benzene: Biomarkers of exposure and nucleic acid oxidation and their modulation by polymorphic xenobiotic metabolizing enzymes", TOXICOLOGY LETTERS, ELSEVIER BIOMEDICAL PRESS , AMSTERDAM, NL, vol. 193, no. 3, 1 April 2010 (2010-04-01), pages 229 - 235, XP026925916, ISSN: 0378-4274, [retrieved on 20100125], DOI: 10.1016/J.TOXLET.2010.01.013
- GAN WEI ET AL.: "Age-dependent increases in the oxidative damage of DNA, RNA, and their metabolites in normal and senescence-accelerated mice analyzed by LC-MS/MS: Urinary 8-oxoguanosine as a novel biomarker of aging", FREE RADICAL BIOLOGY & MEDICINE, vol. 52, no. 9, 17 February 2012 (2012-02-17), pages 1700 - 1707, XP002682476
- ZHAO CONG: "an Observational Study od Urinary 8-oxo-Gsn Level and its Clinical significance in Patients with Coronary Heart Disease", CHINESE JOURNAL OF CARDIOVASCULAR MEDICINE, vol. 21, no. 3, 31 December 2016 (2016-12-31), pages 191 - 196, XP009520872, DOI: 10.3969/j.issn.1007-5410.2016.03.005

## Description

### Technical Field

The present invention relates to a method for health assessment.

More specifically the present invention relates to the use of ratio of free 8-oxoGsn/creatinine in urine as a new health assessment indicator in clinic (i.e., using the ratio of them as an assessment indicator), and belongs to the field of medical examination.

### Background

Lifestyles, environmental factors and diseases affect people's health all the time. Due to the lack of physical exercise, diseases such as cardiovascular, cerebrovascular, gastrointestinal system, immune system and cervical spine diseases may occur; using computer for a long time may cause vision loss, joint damage, radiation injury, head and shoulder pain, autonomic nerve disorder, depression, arteriosclerotic psychosis, etc.; water pollution, air pollution, automobile exhaust pollution and the like are harmful to the human body, and long-term use of food additives, antibiotics and the like will also adversely affect physical health.

At present, the assessment of individual health status is mostly limited to the scope of medical tests, and multiple medical tests are generally required to obtain the evaluation results of overall health status of the tested individual, which have the disadvantages of time-consuming and high testing costs, and are not suitable for the rapid analysis of large quantities of samples, nor for diagnosis and assessment of overall health. In addition, there is a lack of clinical indicators to indicate whether an individual is in an unhealthy status when the body does not show a "sub-healthy" status with pathological changes, such as fatigue, weak, decreased immunity and resistance, easy colds, shortness of breath during exercise, sweating, backache, leg pain, loss of appetite and so on, as well as mental malaise, depressed mood, sluggish response, insomnia and dreaminess, attention-deficit disorder, memory loss, dysphoria, anxiety, and frightening, etc.

Recent studies have shown that oxidative damage to RNA is a new pathogenic mechanism that plays an important role in aging, diabetes and other diseases, and has received more and more attention. 8-Oxidized guanosine (8-oxo-Gsn) is an oxidation product of guanosine in RNA and can be used as a biomarker to indicate the level of RNA oxidative damage in the body. Compared to other tissues and body fluid samples, 8-oxo-Gsn in urine changes more rapidly after oxidative damage and is more easily detected. Chinese patent ZL 201110309531 discloses a method for detecting 8-oxoGsn (8-oxo-Gsn) in urine of a mammal, in which the kit, detection system and detection method of the invention can quickly and easily determine the concentration of 8-oxoGsn, showing good specificity, high sensitivity, accurate and reliable results. The 8-oxoGsn detection technology of the invention is currently an advanced biological information detection technology in the world, and has been successfully applied to the evaluation of human aging degree and early warning of aging-related diseases.

In ANDREOLI R ET AL: "Reference ranges of urinary biomarkers of oxidized guanine in (2'-deoxy)ribonucleotides and nucleic acids", FREE RAD BIO, vol. 50, no. 2, 15 January 2011, pages 254-261, a method for detecting the oxidative stress of patients vs. their healthy counterparts is disclosed, wherein the concentrations of 8-oxoGsn and 8-oxodGsn are determined relative to the subjects creatinine level in urine, and the resulting data were compare to reference data originating from healthy subjects.

Given the high sensitivity of 8-oxoGsn and the relevance of increased level thereof to various diseases, it is necessary to study the relationship between 8-oxoGsn level in urine and diseases. At present, there is no report on the relationship between the interval of the ratio of 8-oxoGsn concentration to creatinine concentration and the health status, and existing studies have not shown that 8-oxoGsn can be used as a health marker.

### Contents of the Invention

The problem to be solved by the present invention is to provide a novel health condition marker for a mammal (including but not limited to a human), and a reagent for detecting the marker.

The invention is directed to subject matter as defined in the claims.

The present invention has been completed based on the following findings of the applicant, that is, there is a detectable and evaluable correspondence between the ratio of 8-oxoGsn concentration to creatinine concentration (8-oxoGsn/creatinine) in a body fluid of a mammal (including but not limited to a human) and the health status of the mammal, and this ratio can be used as a new marker of mammalian health.

To achieve the above purpose, the present invention adopts the following technical solutions.

A first aspect of the present invention provides a use of a reagent for identifying a marker in manufacture of a reagent for detecting a health status of a mammal, comprising:
(1) detecting a marker in a sample of a subject, wherein the marker is a ratio of 8-oxoGsn concentration to creatinine concentration;
(2) comparing the marker with a reference, wherein a difference of the marker compared with the reference is used to evaluate a health status of the subject.

The sample is a body fluid.

The body fluid includes peripheral blood, serum, plasma, synovial fluid, aqueous humor, breast milk, semen, prostate fluid, sweat, tear, pleural effusion, ascites fluid, pericardial fluid, chyle, bile, interstitial fluid, menstrual blood, vomitus, vaginal secretion, mucosal secretion, pancreatic juice, bronchopulmonary suction fluid, blastocyst cavity fluid, umbilical cord blood, urine, cerebrospinal fluid, saliva, lymph fluid and excreta.

The body fluid is preferably urine, peripheral blood, serum, or plasma. Most preferred is urine of a mammal.

For a plasma sample, proteins are first precipitated with acetonitrile, the supernatant is blow dried with nitrogen, redissolved with a mobile phase, and the amount of 8-oxoGsn is detected by HPLC-MS/MS. The concentration of 8-oxoGsn is directly derived from an 8-oxoGsn standard curve.

For a blood sample, HPLC eluent is usually used in gradient elution to obtain better separation results. But this necessarily increases the complexity of detection. At the same time, the treatment of sample must be more complicated, resulting in longer testing time and increased cost. Therefore, from the perspective of convenience and economy, a urine sample is most preferred.

The level of 8-oxoGsn is the ratio of 8-oxoGsn concentration to creatinine concentration in a sample. The creatinine concentration of urine sample is measured simultaneously to correct the effect of drinking water on the 8-oxoGsn concentration in urine.

The reference is any one or both of the following:
(1) an interval value of 8-oxoGsn level of a healthy mammal;
(2) an interval value of ratio of 8-oxoGsn concentration to creatinine concentration of a healthy mammal.

An interval value is 95% percentile. The 0-95 percentile of healthy subjects is used as a normal reference interval.

The mammal according to the present invention is preferably a human, but may also be other mammal, such as cat, dog, horse, and the like.

The method for determining 8-oxoGsn in a mammal belongs to the prior art. According to a preferred embodiment, an 8-oxoGsn standard is first used to prepare a standard curve, so that the concentration of 8-oxoGsn in a sample can be determined more quickly and accurately. The concentration range of the standard curve can be adjusted according to the different samples to be measured.

According to a more preferred embodiment, an internal standard is added to the sample to be measured in order to correct the error of measuring instrument. The internal standard may be 8-oxoGsn in which any one or more elements are replaced by isotopes, and preferably at least one of N and C is replaced by their corresponding isotopes. For example, ¹⁴N can be replaced by ¹⁵N, and ¹²C can be replaced by ¹³C. It is possible to replace only one element, or replace two or more elements. In one test, the concentration of internal standard in each sample is the same.

It is further disclosed a use of a reagent for detecting 8-oxoGsn in manufacture of a product (including but not limited to a kit) for evaluating a health status of a mammal.

The reagent for detecting 8-oxoGsn is a reagent for determining the concentration of 8-oxoGsn.

The mammal is a human.

It is further disclosed a use of a reagent for detecting 8-oxoGsn concentration and creatinine concentration in manufacture of a product (including but not limited to a kit) for evaluating a health status of a mammal.

By using the kit, the 8-oxoGsn concentration and creatinine concentration in a sample of a subject are measured, the ratio of the 8-oxoGsn concentration to the creatinine concentration is calculated, and the ratio is compared with a reference to evaluate the health status of the subject who is the source of the sample to be tested.

The reference is an interval (95% percentile) of 8-oxoGsn level in a healthy mammal.

The reference is an interval (95% percentile) of the ratio of 8-oxoGsn concentration to creatinine concentration in a healthy mammal.

It is further disclosed a kit for detecting a health status of a mammal, comprising a reagent for detecting 8-oxoGsn concentration and a reagent for detecting creatinine concentration.

The mammal is a human.

The kit comprises 8-oxoGsn standard, 10mM ammonium acetate, methanol, 1 to 2000 pg/µL isotope internal standard [¹³C,¹⁵N₂]8-oxo-dGsn, and 1 to 2000 pg/µL isotope internal standard [¹⁵N₅]8-oxoGsn; preferably, the isotope internal standards have a concentration of 100 to 800 pg/µL.

The 10 mM ammonium acetate and methanol are 70% methanol + 30% 10 mM ammonium acetate solution, and the solution after mixing has a pH of 3.5 to 4.0. That is, the working solution in this kit is prepared from methanol and 10 mM ammonium acetate solution in a volume ratio of 7: 3.

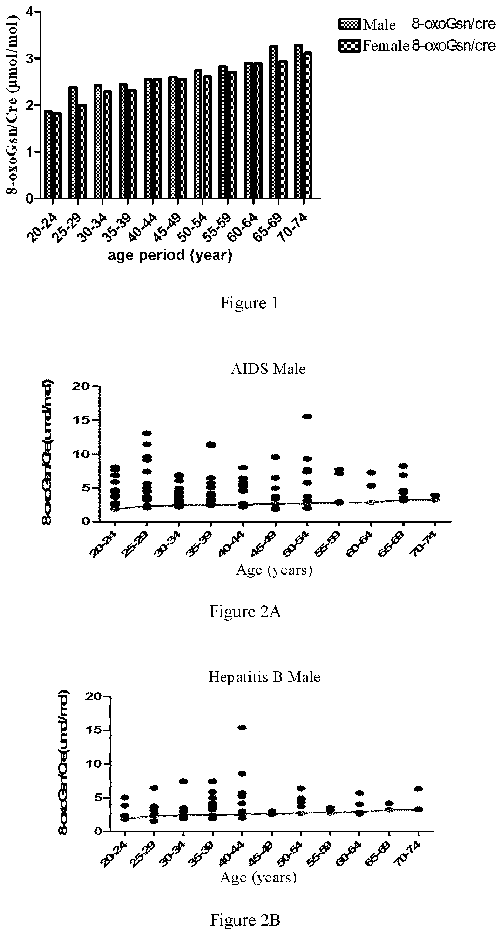

We established a reference interval of 8-oxoGsn for urine samples from normal population in Beijing, China by using the isotope dilution high performance liquid chromatography mass spectrometry technology (the method was the same as Chinese patent ZL201110309531.5, "Application of kit, detection system and 8-oxoGsn for assessment of aging degree of mammals"), as shown in Figure 1 and Table 1. By studying a large size of samples, it was found that normal humans between the ages of 20 and 74, regardless of gender, had a positive correlation between the 8-oxoGsn content in their urine and their age, which could be used as an age-related indicator.

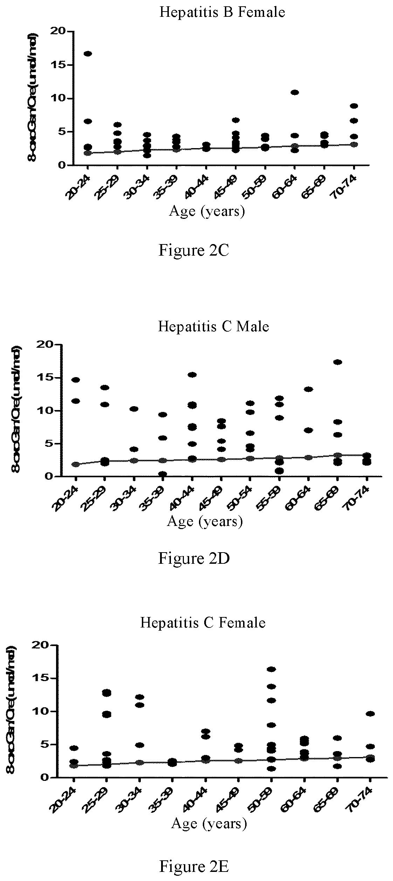

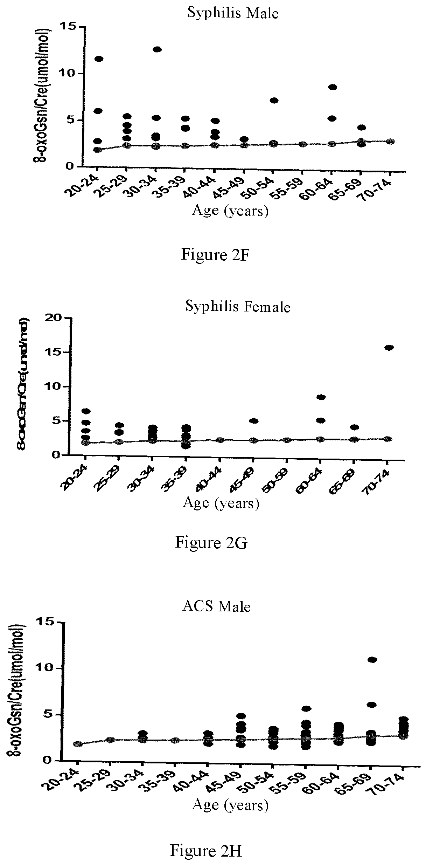

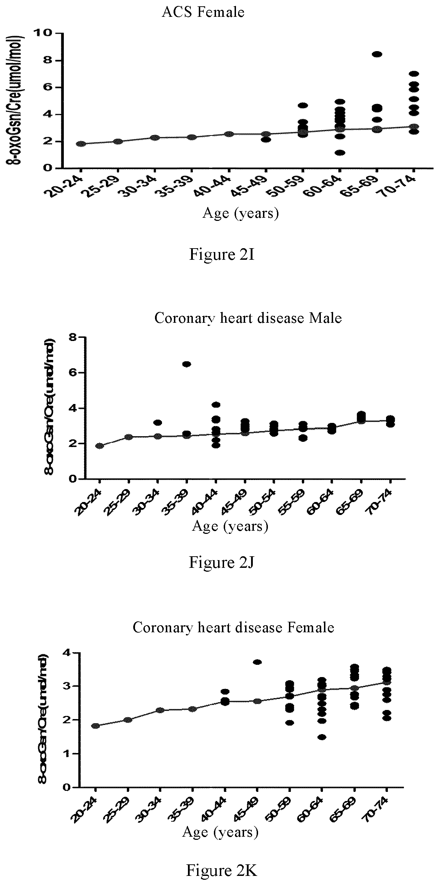

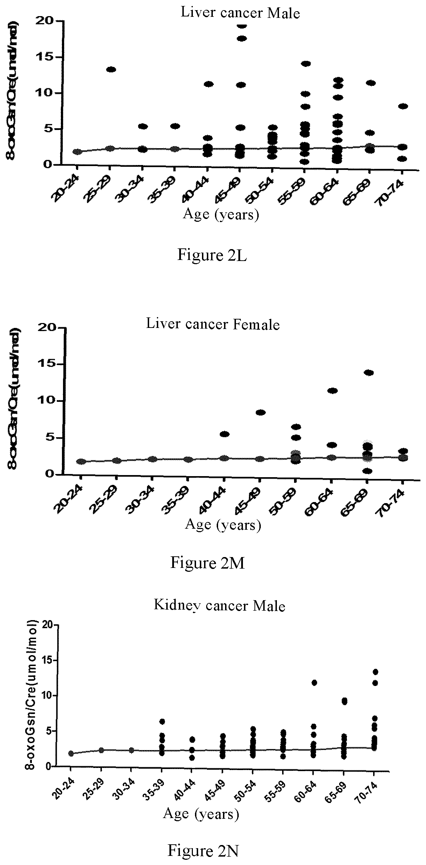

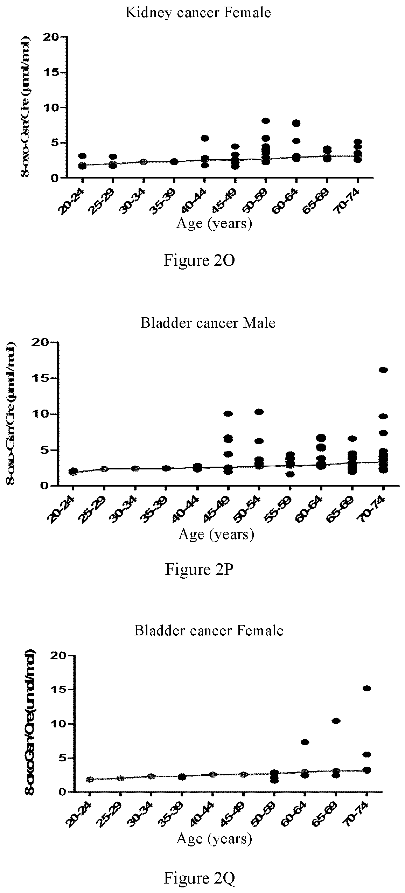

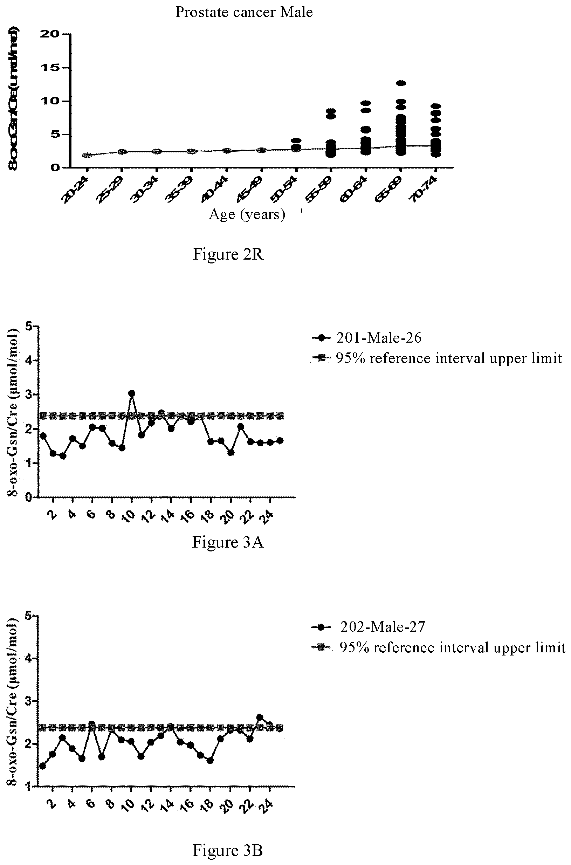

By using the same method, we determined 8-oxoGsn in urine samples of patients of seven diseases (AIDS, hepatitis B, hepatitis C, syphilis, liver cancer, coronary heart disease, acute coronary syndrome), compared the results of the seven diseases with the above reference interval of 8-oxoGsn for normal humans, and found that the 8-oxoGsn contents in the urine samples of the patients of various diseases increased significantly as compared with the normal humans. See Table 2 and Figure 2 for details. At least 60% of the patients of each disease had higher values than the interval for normal humans. In particular, for the bacterial and viral infectious diseases, the proportion of number of the patients having a value higher than the interval for normal humans was as high as about 90%, while chronic diseases such as syphilis, AIDS, hepatitis B, etc., come next, which proved that the increase of 8-oxoGsn was correlated with diseases.

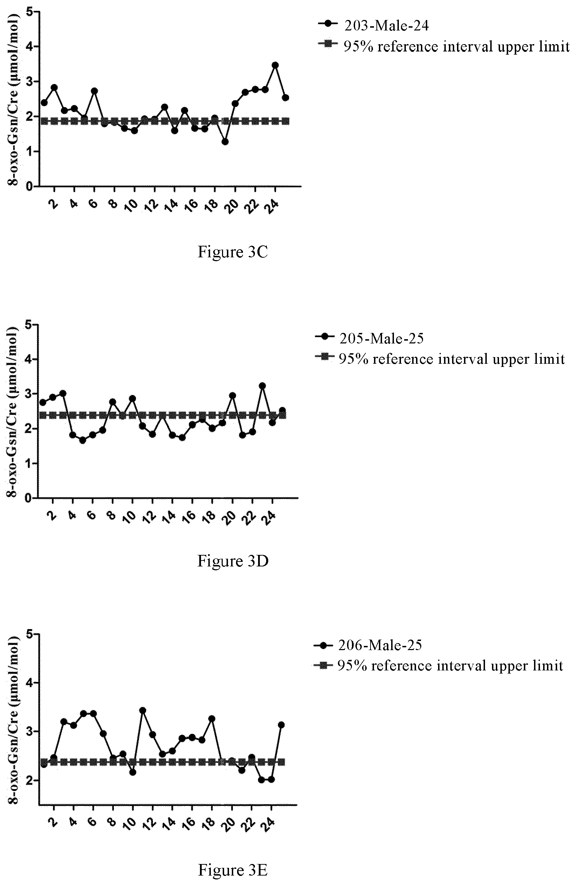

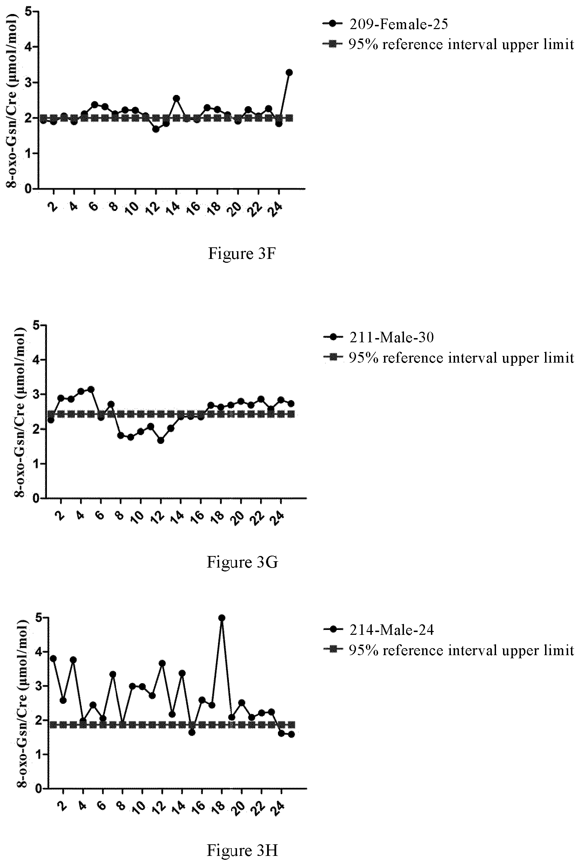

Further, we recruited 8 healthy volunteers aged 20-30, each of them had to collect urine 25 times within 2 months, and each time the urine was collected with a urine cup, immediately transferred to a 5ml urine tube and stored in a refrigerator at -80 °C, and the physical conditions of the day, such as whether there was strenuous exercise, physical discomfort, insomnia, high work intensity and other factors, were recorded in the corresponding record manual (see Table 3 for details), which were used to screen the specific influencing factors causing the increase of 8-oxoGsn level in normal humans. When the final test results of each person's urine samples were compared with the corresponding upper limit of the 95th percentile of the reference interval for normal humans, it was found that most of the time points where the values were above the upper limit of the interval for normal humans corresponded to the special statuses of the day, such as illness, excessive fatigue, staying up late, strenuous exercise, etc. The above results fully reflect the high sensitivity of the 8-oxoGsn level in urine, that is, as long as a normal healthy person feels an uncomfortable symptom, especially a state of disease, whose level would increase correspondingly, so that this may reflect a health status of a human to a certain extent. In addition, it was found in this experiment that the level of RNA oxidation in a human who exercised regularly was significantly lower than that in those who did not exercise, for example, as shown in Figure 3, the healthy humans Nos. 201 and 202 basically maintained their 8-oxoGsn levels in urine below the upper limit of the 95th percentile of the reference interval for normal humans.

In addition, we also tested the level of nucleic acid oxidation in the plasma and urine of patients with chronic kidney disease (CKD). From Table 4, it could be found that the level of RNA oxidation was positively correlated with the severity of the disease, so that the increased 8-oxoGsn ratio in plasma and urine might be a new assessment indicator for advanced kidney disease.

Through a large number of experimental studies, the present invention obtained the value interval of 8-oxoGsn in subjects of different ages at health status, and corresponding data at different health statuses such as psychological stress status, sub-health status, chronic disease status, acute disease status, cancerous disease status, viral disease status and the like. Through scientific and in-depth analysis and research on these data, the present invention has found for the first time the correlation between 8-oxoGsn and the overall health of human, in which the value of 8-oxoGsn and the health status show a significant positive correlation, so that a valuable health status assessment of a subject can be carried out by only measuring 8-oxoGsn of the body. The above research results show that the 8-oxoGsn in urine is not only a useful indicator for evaluating oxidative stress and aging of the body, but also a new clinical health evaluation indicator.

The present invention has wide application prospects, and can be used for detection and early warning of diseases, psychological evaluation of students, troops and special working groups, supporting detection of the disease status of tumor patients and so on, and is particularly suitable for epidemiological censuses, for health screening of humans at a certain area or a certain age group, for assessment of effects of external factors on the health status of the population, and for guiding people to change their lifestyles or receive medical services to improve their health. The present invention adopts a simple clinical physical examination method to conduct a comprehensive health status assessment on a subject, can timely and quickly grasp people's health status and health rules, can be used in the field of health service management for sub-health detection and early warning, disease treatment and effect observation, and detection of psychological and emotional state, and have significant advantages over conventional medical detection methods.

The following describes the present invention in detail with reference to the examples and the drawings, but is not a limitation on the present invention. Any equivalent replacement in the art made in accordance with the disclosure of the present invention belongs to the protection scope of the present invention.

### Brief Description of the Drawings

Figure 1 shows the creatinine-corrected 8-oxoGsn/cre values in the urine samples of normal males and females aged 20-74 years.
Figure 2A shows a comparison of the urine 8-oxoGsn/cre level of male patients with HIV with the upper limit of the 95th percentile of the reference interval for normal humans.
Figure 2B shows a comparison of the urine 8-oxoGsn/cre level of male patients with hepatitis B with the upper limit of the 95th percentile of the reference interval for normal humans.
Figure 2C shows a comparison of the urine 8-oxoGsn/cre level of female patients with hepatitis B with the upper limit of the 95th percentile of the reference interval for normal humans.
Figure 2D shows a comparison of the urine 8-oxoGsn/cre level of male patients with hepatitis C with the upper limit of the 95th percentile of the reference interval for normal humans.
Figure 2E shows a comparison of the urine 8-oxoGsn/cre level of female patients with hepatitis C with the upper limit of the 95th percentile of the reference interval for normal humans.
Figure 2F shows a comparison of the urine 8-oxoGsn/cre level of male patients with syphilis with the upper limit of the 95th percentile of the reference interval for normal humans.
Figure 2G shows a comparison of the urine 8-oxoGsn/cre level of female patients with syphilis with the upper limit of the 95th percentile of the reference interval for normal humans.
Figure 2H shows a comparison of urine 8-oxoGsn/cre levels of male patients with ACS with the upper limit of the 95th percentile of the reference interval for normal humans.
Figure 2I shows a comparison of the urine 8-oxoGsn/cre level of female patients with ACS with the upper limit of the 95th percentile of the reference interval for normal humans.
Figure 2J shows a comparison of the urine 8-oxoGsn/cre level of male patients with coronary heart disease with the upper limit of the 95th percentile of the reference interval for normal humans.
Figure 2K shows a comparison of the urine 8-oxoGsn/cre level of female patients with coronary heart disease with the upper limit of the 95th percentile of the reference interval for normal humans.
Figure 2L shows a comparison of the urine 8-oxoGsn/cre level of male patients with liver cancer with the upper limit of the 95th percentile of the reference interval for normal humans.
Figure 2M shows a comparison of the urine 8-oxoGsn/cre level of female patients with liver cancer with the upper limit of the 95th percentile of the reference interval for normal humans.
Figure 2N shows a comparison of the urine 8-oxoGsn/cre level of male patients with kidney cancer with the upper limit of the 95th percentile of the reference interval for normal humans.
Figure 2O shows a comparison of the urine 8-oxoGsn/cre level of female patients with kidney cancer with the upper limit of the 95th percentile of the reference interval for normal humans.
Figure 2P shows a comparison of the urine 8-oxoGsn/cre level of male patients with bladder cancer with the upper limit of the 95th percentile of the reference interval for normal humans.
Figure 2Q shows a comparison of the urine 8-oxoGsn/cre levels of female patients with bladder cancer with the upper limit of the 95th percentile of the reference interval for normal humans.
Figure 2R shows a comparison of the urine 8-oxoGsn/cre level of male patients with prostate cancer with the upper limit of the 95th percentile of the reference interval for normal humans.
Figures 3A to 3H show comparisons the 8-oxoGsn/cre levels in 25 urine samples collected from 8 normal humans within 2 months with the upper limit of the 95th percentile of the reference interval for normal humans.

### Examples

### Example 1: Determination of 8-oxoGsn content in urine of normal humans aged 20-74 years

### I. Reagents and methods:

(1) Samples: 5179 urine samples from normal humans, including 2591 males and 2588 females.
(2) Reagents and instruments:
   Reagents: 8-oxidized guanosine (purity > 98%), purchased from ALEXIS Company, the United States; 8-oxidized deoxyguanosine (purity > 98%), purchased from Sigma-Aldrich, the United States; [¹³C, ¹⁵N₂]8-oxidized guanosine (purity > 98%), purchased from Toronto Research Chemical Company, Canada; [¹⁵N₅]8-oxidized deoxyguanosine (purity > 98%), purchased from Cambridge Isotope Laboratory, the United States; creatinine detection kit, purchased from Beijing Jiuqiang Biotechnology Company, China; methanol (HPLC grade), purchased from Merck, Germany; isopropanol (HPLC grade), purchased from Merck, Germany; formic acid (HPLC grade), purchased from Merck, Germany; ammonium acetate (HPLC grade), purchased from Fisher Technology, USA;
   Chromatography column: Agilent SB-Aq, 3.0x100mm, 1.8µm
   Instrument: the instrument used for HPLC-MS/MS analysis was an Agilent LC (1290) tandem mass spectrometer (6490) instrument.
(3) Determination method:
   3ml of middle urine in random urine was collected, evenly divided into 3 parts and placed into 1.5ml EP tubes, and stored in a refrigerator at -80 °C until testing. The treatment of urine samples was as follows:
   1. A urine sample was thawed and centrifuged at 7,500 g for 5 min at 4 °C.
   2. 200 µL of the supernatant was pipetted into a 1.5 ml autoclaved centrifuge tube. 200 µL of working solution A [70% methanol + 30% 10 mM ammonium acetate (PH = 3.7)] was added; and the working solution was used to precipitate proteins present in the urine.
   3. 480 pg/µL isotope internal standard [¹³C, ¹⁵N₂]8-oxo-dGsn and 480 pg/µL isotope internal standard [¹⁵N₅]8-oxoGsn, each 10µL, were added, mixed by votex for 2min; the internal standards were used for the correction of measuring instrument errors.
   4. After being placed in a 37 °C biochemical incubator for 10 minutes, centrifugation was carried out at 12000g and 4 °C for 15 minutes; this step allowed the 8-oxoG combined with the precipitate to be released.
   5. 100ul was taken and added into a disposable intubation tube, loaded into a sample bottle, and subjected to mass spectrometry sequence detection.
   6. The standard 8-oxoG was detected by HPLC-MS/MS, and a standard curve of 8-oxoG concentration versus intensity was prepared. At the same time, the intensity of 8-oxoG in the sample was detected by HPLC-MS/MS, and the concentration of 8-oxoG in the sample was calculated against the standard curve.
   7. The creatinine concentration in the sample was measured with a Hitachi biochemical detector; which was used to correct the effect of different dilution levels of urine on the results.

### II. Results

See Figure 1 and Table 1 (creatinine-corrected mean values and percentiles of 8-oxoGsn in the reference population). A large sample study found that, for normal humans aged 20 to 74, regardless of gender, the 8-oxoGsn content had a positive correlation with the age, and could be used as an age-related indicator. Compared with normal humans, the level of 8-oxoGsn in patients with diseases was higher than that in normal humans, and the higher values, the more meaningful. Therefore, the 0-95 percentile of each age group was used as the reference interval for normal humans (see Table 1 for 0-95% reference interval for each age group).

### Example 2: Determination of 8-oxoGsn content in urine of patients of seven diseases

### I. Reagents and methods:

(1) Samples: 111 urine samples from AIDS patients, 115 urine samples from hepatitis B patients, 104 urine samples from hepatitis C patients, 79 urine samples from syphilis patients, 90 urine samples from liver cancer patients, 119 urine samples from patients with coronary heart disease, and 98 urine samples from ACS patients were collected.
(2) Reagents and instruments:
   Reagents: 8-oxidized guanosine (purity > 98%), purchased from ALEXIS Company, the United States; 8-oxidized deoxyguanosine (purity > 98%), purchased from Sigma-Aldrich, the United States; [¹³C, ¹⁵N₂]8-oxidized guanosine (purity > 98%), purchased from Toronto Research Chemical Company, Canada; [¹⁵N₅]8-oxidized deoxyguanosine (purity > 98%), purchased from Cambridge Isotope Laboratory, the United States; creatinine detection kit, purchased from Beijing Jiuqiang Biotechnology Company, China; methanol (HPLC grade), purchased from Merck, Germany; isopropanol (HPLC grade), purchased from Merck, Germany; formic acid (HPLC grade), purchased from Merck, Germany; ammonium acetate (HPLC grade), purchased from Fisher Technology, USA;
   Chromatography column: Agilent SB-Aq, 3.0x100mm, 1.8µm
   Instrument: the instrument used for HPLC-MS/MS analysis was an Agilent LC (1290) tandem mass spectrometer (6490) instrument.
(3) Determination method:
   3ml of middle urine in random urine was collected, evenly divided into 3 parts and placed into 1.5ml EP tubes, and stored in a refrigerator at -80 °C until testing. The treatment of urine samples was as follows:
   1. A urine sample was thawed and centrifuged at 7,500 g for 5 min at 4 °C.
   2. 200 µL of the supernatant was pipetted into a 1.5 ml autoclaved centrifuge tube. 200 µL of working solution A [70% methanol + 30% 10 mM ammonium acetate (PH = 3.7)] was added; and the working solution was used to precipitate proteins present in the urine.
   3. 480 pg/µL isotope internal standard [¹³C, ¹⁵N₂]8-oxo-dGsn and 480 pg/µL isotope internal standard [¹⁵N₅]8-oxoGsn, each 10µL, were added, mixed by votex for 2min; the internal standards were used for the correction of measuring instrument errors.
   4. After being placed in a 37 °C biochemical incubator for 10 minutes, centrifugation was carried out at 12000g and 4 °C for 15 minutes; this step allowed the 8-oxoG combined with the precipitate to be released.
   5. 100ul was taken and added into a disposable intubation tube, loaded into a sample bottle, and subjected to mass spectrometry sequence detection.
   6. The standard 8-oxoG was detected by HPLC-MS/MS, and a standard curve of 8-oxoG concentration versus intensity was prepared. At the same time, the intensity of 8-oxoG in the sample was detected by HPLC-MS/MS, and the concentration of 8-oxoG in the sample was calculated against the standard curve.
   7. The creatinine concentration in the sample was measured with a Hitachi biochemical detector; which was used to correct the effect of different dilution levels of urine on the results.

### II. Results

The results were shown in Figure 2 and Table 2 (the numbers and percentages of patients with various diseases that were of above the 95th percentile of the reference interval for normal humans), and for the 8-oxoGsn in urine samples of patients with seven diseases (AIDS, hepatitis B, hepatitis C, syphilis, liver cancer, coronary heart disease, acute coronary syndrome), a comparative study was performed between the results of the seven diseases and the reference interval of 8-oxoGsn for normal humans, and it was found that the levels of 8-oxoGsn in urine samples of patients with various diseases were significantly increased compared to that of the normal humans (see Table 2 and Figure 2 for details), and at least 60% of the patients of each disease had higher values than the interval for normal humans. In particular, for the bacterial and viral infectious diseases, the proportion of number of patients having a value higher than the interval for normal humans was as high as about 90%, while chronic diseases such as syphilis, AIDS, hepatitis B, etc., come next, which proved that the increase of 8-oxoGsn was correlated with the diseases.

### Example 3: Determination of 8-oxoGsn content in urine of healthy volunteers

### I. Reagents and methods:

(I) Samples: collected from a total of 8 healthy volunteers, and urine was collected for 25 times from each of the volunteers.

### (2) Reagents and instruments:

Reagents: 8-oxidized guanosine (purity > 98%), purchased from ALEXIS Company, the United States; 8-oxidized deoxyguanosine (purity > 98%), purchased from Sigma-Aldrich, the United States; [¹³C, ¹⁵N₂]8-oxidized guanosine (purity > 98%), purchased from Toronto Research Chemical Company, Canada; [¹⁵N₅]8-oxidized deoxyguanosine (purity > 98%), purchased from Cambridge Isotope Laboratory, the United States; creatinine detection kit, purchased from Beijing Jiuqiang Biotechnology Company, China; methanol (HPLC grade), purchased from Merck, Germany; isopropanol (HPLC grade), purchased from Merck, Germany; formic acid (HPLC grade), purchased from Merck, Germany; ammonium acetate (HPLC grade), purchased from Fisher Technology, USA;
Chromatography column: Agilent SB-Aq, 3.0x100mm, 1.8µm
Instrument: the instrument used for HPLC-MS/MS analysis was an Agilent LC (1290) tandem mass spectrometer (6490) instrument.

### (3) Determination method:

3ml of middle urine in random urine was collected, evenly divided into 3 parts and placed into 1.5ml EP tubes, and stored in a refrigerator at -80 °C until testing. The treatment of urine samples was as follows:
1. A urine sample was thawed and centrifuged at 7,500 g for 5 min at 4 °C.
2. 200 µL of the supernatant was pipetted into a 1.5 ml autoclaved centrifuge tube. 200 µL of working solution A [70% methanol + 30% 10 mM ammonium acetate (PH = 3.7)] was added; and the working solution was used to precipitate proteins present in the urine.
3. 480 pg/µL isotope internal standard [¹³C, ¹⁵N₂]8-oxo-dGsn and 480 pg/µL isotope internal standard [¹⁵N₅]8-oxoGsn, each 10µL, were added, mixed by votex for 2min; the internal standards were used for the correction of measuring instrument errors.
4. After being placed in a 37 °C biochemical incubator for 10 minutes, centrifugation was carried out at 12000g and 4 °C for 15 minutes; this step allowed the 8-oxoG combined with the precipitate to be released.
5. 100ul was taken and added into a disposable intubation tube, loaded into a sample bottle, and subjected to mass spectrometry sequence detection.
6. The standard 8-oxoG was detected by HPLC-MS/MS, and a standard curve of 8-oxoG concentration versus intensity was prepared. At the same time, the intensity of 8-oxoG in the sample was detected by HPLC-MS/MS, and the concentration of 8-oxoG in the sample was calculated against the standard curve.
7. The creatinine concentration in the sample was measured with a Hitachi biochemical detector; which was used to correct the effect of different dilution levels of urine on the results.

### II. Results

The results were shown in Figure 3 and Table 3 (the physical discomfort records of the 8 healthy volunteers on the days for collecting 25 urine samples were assessed), and when the final test results of each volunteer's urine were compared with the corresponding upper limit of the 95th percentile of the reference interval for normal humans, it was found that most of the time points where the values were above the upper limit of the interval for normal humans corresponded to the special statuses of the day, such as illness, excessive fatigue, staying up late, strenuous exercise and the like. The above results fully reflected the high sensitivity of the level of 8-oxoGsn in urine, that was, as long as a normal healthy person felt an uncomfortable symptom, especially a state of disease, whose level would increase correspondingly, so that this could reflect a health status of a human to a certain extent. In addition, it was found in this experiment that the level of RNA oxidation in a human who exercised regularly was significantly lower than those who did not exercise, for example, as shown in Figure 3, the healthy humans Nos. 201 and 202 basically maintained their 8-oxoGsn levels in urine below the upper limit of the 95th percentile of the reference interval for normal humans.

### Example 4:

### I. Reagents and methods:

(I) Test population and sample size: plasma and urine samples were collected from a total of 146 patients with chronic kidney diseases, which included 30 patients with phase I chronic kidney disease, 30 patients with phase II chronic kidney disease, 31 patients with phase III chronic kidney disease, 30 patients with phase IV chronic kidney disease, and 25 patients with phase V chronic kidney disease.
   (2) Reagents and instruments, comprising:
      Reagents: 8-oxidized guanosine (purity > 98%), purchased from ALEXIS Company, the United States; 8-oxidized deoxyguanosine (purity > 98%), purchased from Sigma-Aldrich, the United States; [¹³C, ¹⁵N₂]8-oxidized guanosine (purity > 98%), purchased from Toronto Research Chemical Company, Canada; [¹⁵N₅]8-oxidized deoxyguanosine (purity > 98%), purchased from Cambridge Isotope Laboratory, the United States; creatinine detection kit, purchased from Beijing Jiuqiang Biotechnology Company, China; methanol (HPLC grade), purchased from Merck, Germany; isopropanol (HPLC grade), purchased from Merck, Germany; formic acid (HPLC grade), purchased from Merck, Germany; ammonium acetate (HPLC grade), purchased from Fisher Technology, USA;
      Chromatography column: Agilent SB-Aq, 3.0x100mm, 1.8µm
      Instrument: the instrument used for HPLC-MS/MS analysis was an Agilent LC (1290) tandem mass spectrometer (6490) instrument.
   (3) Determination method: urine and/or plasma samples could be selected for detection

Detection of urine sample: 3ml of middle urine in random urine was collected, evenly divided into 3 parts and placed into 1.5ml EP tubes, and stored in a refrigerator at -80 °C until testing. The treatment of urine samples was as follows:
1. A urine sample was thawed and centrifuged at 7,500 g for 5 min at 4 °C.
2. 200 µL of the supernatant was pipetted into a 1.5 ml autoclaved centrifuge tube. 200 µL of working solution A [70% methanol + 30% 10 mM ammonium acetate (PH = 3.7)] was added; and the working solution was used to precipitate proteins present in the urine.
3. 480 pg/µL isotope internal standard [¹³C, ¹⁵N₂]8-oxo-dGsn and 480 pg/µL isotope internal standard [¹⁵N₅]8-oxoGsn, each 10µL, were added, mixed by votex for 2min; the internal standards were used for the correction of measuring instrument errors.
4. After being placed in a 37 °C biochemical incubator for 10 minutes, centrifugation was carried out at 12000g and 4 °C for 15 minutes; this step allowed the 8-oxoG combined with the precipitate to be released.
5. 100ul was taken and added into a disposable intubation tube, loaded into a sample bottle, and subjected to mass spectrometry sequence detection.
6. The standard 8-oxoG was detected by HPLC-MS/MS, and a standard curve of 8-oxoG concentration versus intensity was prepared. At the same time, the intensity of 8-oxoG in the sample was detected by HPLC-MS/MS, and the concentration of 8-oxoG in the sample was calculated against the standard curve.
7. The creatinine concentration in the sample was measured with a Hitachi biochemical detector; which was used to correct the effect of different dilution levels of urine on the results.

Determination of plasma samples: 3ml of plasma was collected from a patient, evenly aliquoted and loaded into 1.5ml EP tubes, and stored at -80 °C in a refrigerator until testing. The treatment of plasma samples was as follows:
1. The collected plasma was taken from the -80 °C refrigerator and thawed on ice, mixed well and centrifuged at 14000g and 4 °C for 10 minutes.
2. 300 µL was taken and added with two internal standards, 300 pg/µL and 10 µL for each. The internal standards were used to correct errors of the measuring instrument.
3. After being mixed well with three times volume acetonitrile of 900 µL, vortex was performed for approximately 1 minute, so that the proteins present in the plasma were precipitated with acetonitrile.
4. After centrifugation at 14,000g for 20 minutes at 4 °C, a quantitative volume of 1ml supernatant was taken and blow dried with nitrogen.
5. After being redissolved with 100 µL of mobile phase, centrifugation was carried out at 12000g for 20 minutes at 4 °C.
6. 85 µL was taken and placed into a disposable intubation tube, loaded into a sample bottle, and subjected to mass spectrometry sequence detection.

### II. Results

The results were shown in Table 4 (plasma, urine creatinine and nucleic acid oxidation levels of patients with chronic kidney diseases). From Table 4, it could be found that the RNA oxidation level was positively correlated with the severity of the disease, so that the increased 8-oxoGsn ratio in plasma and urine could be a new indicator for advanced kidney diseases.

### Example 5: Reagent for identifying marker 8-oxoGsn and use thereof

### I. Kit composition

1. 8-oxoGsn standard, used to prepare a standard curve of 8-oxoGsn concentration versus intensity.
2. 10mM ammonium acetate and pure methanol: used to prepare a working solution [70% methanol + 30% 10mM ammonium acetate (PH = 3.7)]; which could also be a directly prepared 70% methanol + 30% 10mM ammonium acetate solution (pH was 3.5-4.0), that was, the working solution in this kit was prepared from methanol and 10 mM ammonium acetate solution in a volume ratio of 7: 3.
3. 500 pg/µL isotope internal standard [¹³C, ¹⁵N₂]8-oxo-dGsn and 500 pg/µL isotope internal standard [¹⁵N₅]8-oxoGsn.

### II. Use for determining health status

1. Detecting a marker in a sample of a subject, wherein the marker was level of 8-oxoGsn;
2. The marker was compared with a reference (the 95% confidence interval value for normal humans obtained in Example 1), and the difference of the marker compared with the reference was used to evaluate the health status of the subject.
3. The following explained how to compare the marker with the reference (the 95% reference interval value for normal humans obtained in Example 1) for evaluating the health status of the subject: among the 25 tests for the urine samples collected from the healthy volunteers, the marker concentrations obtained from the 25 urine tests were compared with the upper limit of the 95% reference interval for normal humans in the age group. Above 95% reference interval indicated that the subject was in a state of high oxidative stress. The concentrations of most or all of the 25 urine markers were higher than the 95% reference interval, indicating that the subject was in an unhealthy state with persistent high oxidative stress, and intervention was needed. Based on the physical discomfort records of the subjects (see Table 3), the effects of exogenous and endogenous pathogenic factors on 8-oxoGsn levels in human urine were identified, and the main factors that caused the increase of 8-oxoGsn level were screened, and real-time assessment of human health was performed to achieve the purpose of early warning of human sub-health and disease.

Although the present invention has been described in detail according to specific embodiments, it should be understood that those skilled in the art can readily conceive of changes, variations, or equivalents of these embodiments after gaining an understanding of the foregoing. Accordingly, the scope of the present invention should be evaluated as that of the appended claims.

**Table 1: Means and percentiles of 8-oxoGsn in reference population after creatinine correction**

| Age group | Male | | | | | Female | | | | | | |
|---|---|---|---|---|---|---|---|---|---|---|---|---|
| | No. | Creatinine | 5th | Median (IQR) | 95th | Mean ± standard deviation | No. | Creatinine | 5th | Median (IQR) | 95th | Mean ± standard deviation |
| 20-24 | 121 | 19442(8878) | 1.50 | 1.69(0.14) | 1.87 | 1.7±0.11 | 121 | 15219(17118) | 1.48 | 1.68(0.15) | 1.82 | 1.67±0.1 |
| 25-29 | 125 | 18501(9586) | 1.14 | 1.72(0.45) | 2.38 | 1.7±0.32 | 124 | 12996(10642) | 1.18 | 1.60(0.44) | 2.00 | 1.6±0.25 |
| 30-34 | 130 | 15756(7010) | 1.19 | 1.93(0.37) | 2.43 | 1.89±0.34 | 130 | 12518(9591) | 1.31 | 1.86(0.40) | 2.29 | 1.84±0.28 |
| 35-39 | 120 | 14828(6971) | 1.96 | 2.23(0.19) | 2.45 | 2.23±0.14 | 131 | 12005(7572) | 1.8 | 2.04(0.21) | 2.32 | 2.03±0.14 |
| 40-44 | 125 | 16003(8118) | 2.09 | 2.32(0.15) | 2.55 | 2.33±0.13 | 122 | 10777(8401) | 1.44 | 2.12(0.42) | 2.55 | 2.06±0.33 |
| 45-49 | 132 | 15942(7864) | 2.16 | 2.45(0.2) | 2.61 | 2.43±0.14 | 133 | 11229(7000) | 2.07 | 2.31(0.18) | 2.56 | 2.31±0.13 |
| 50-54 | 133 | 15455(8087) | 1.91 | 2.43(0.27) | 2.74 | 2.38±0.25 | 127 | 10058(8086) | 2.09 | 2.49(0.24) | 2.61 | 2.44±0.17 |
| 55-59 | 128 | 14877(10014) | 2.2 | 2.58(0.17) | 2.83 | 2.55±0.16 | 137 | 9542(6640) | 2.21 | 2.51(0.17) | 2.70 | 2.5±0.13 |
| 60-64 | 129 | 12808(8747) | 2.45 | 2.71(0.15) | 2.89 | 2.68±0.12 | 120 | 10355(7945) | 2.52 | 2.71(0.12) | 2.90 | 2.71±0.09 |
| 65-69 | 132 | 12872(6817) | 2.12 | 2.81(0.35) | 3.26 | 2.75±0.32 | 125 | 9643(6428) | 2.6 | 2.77(0.19) | 2.94 | 2.78±0.11 |
| 70-74 | 121 | 12842(6522) | 2.77 | 3.02(0.18) | 3.28 | 3.02±0.16 | 128 | 8934(7056) | 2.5 | 2.84(0.27) | 3.11 | 2.83±0.17 |

Data were grouped by gender, and the concentration of creatinine was expressed as µmol/L.

**Table 2: Number and percentage of various diseases above the 95th percentile of the reference interval for normal humans**

| Disease | 8-oxoGsn | | | |
|---|---|---|---|---|
| | Male | Female | Number | Total number |
| AIDS | 90(90%) | | 90(90%) | 100 |
| Hepatitis B | 43(85%) | 43(85%) | 86(86%) | 100 |
| Hepatitis C | 37(74%) | 45(90) | 82(82%) | 100 |
| Syphilis | 37(95%) | 38(91%) | 75 (93%) | 81 |
| Acute coronary syndrome | 56(71%) | 26(79%) | 82(73%) | 112 |
| Coronary heart disease | 38(75%) | 26(52%) | 64(64%) | 100 |
| Liver cancer | 49 (69%) | 17 (81%) | 66(72%) | 92 |
| Kidney cancer 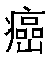 | 63(76%) | 32(70%) | 95(74%) | 129 |
| Bladder cancer | 42(75%) | 5 (45%) | 47(70%) | 67 |
| Prostate | 63(72%) | | 63(72%) | 88 |

**Table 3: Physical discomfort records on the days of 25 urine health assessments for 8 healthy volunteers**

| Time of collecting urine sample | Number of normal humans | | | | | | | |
|---|---|---|---|---|---|---|---|---|
| | 201 | 202 | 203 | 205 | 206 | 209 | 211 | 214 |
| 1 | | | | Work hard | | | | Diarrhea and insomnia |
| 2 | | | | | | | Mood swings | Slightly diarrhea, strenuous exercise |
| 3 | | | | Cold | Insomnia | | Stay-up late | |
| 4 | | | | | Insomnia | | Insomnia | |
| 5 | | | | | Diarrhea | | Stay-up late | |
| 6 | | Strenuous exercise | | | | | | |
| 7 | | | | Cold and taking cold medicine | Diarrhea | | Stay-up late | |
| 8 | | | | Cold and taking cold medicine | | | | |
| 9 | | | | Cold and taking cold medicine | | | | |
| 10 | Diarrhea | | | Cold and taking cold medicine | | | | |
| 11 | | | | | | | | |
| 12 | | | | | | | | |
| 13 | | | | | | | | |
| 14 | | Insomnia | | | | | | Cold |
| 15 | | | | | | | Stay-up late | |
| 16 | | | | | | | Stay-up late | |
| 17 | | | | | Cold | | Stay-up late | |
| 18 | | | | | Cold | | Stay-up late | Cold and strenuous exercise |
| 19 | | | | | | | Stay-up late | Cold |
| 20 | | | | | | | Stay-up late | |
| 21 | | | Anxiety | | | | Stay-up late | |
| 22 | | | Anxiety | | | | Stay-up late | Anxiety and fatigue |
| 23 | | Strenuous exercise | Anxiety | | | | Stay-up late | Anxiety and fatigue |
| 24 | | Strenuous exercise | Anxiety | | | | Stay-up late | |
| 25 | | Strenuous exercise | Anxiety | | | Stomach pain | Stay-up late | |

**Table 4: Plasma, urine creatinine, and nucleic acid oxidation levels in patients with chronic kidney disease**

| | CKD 1 | CKD 2 | CKD 3 | CKD 4 | CKD 5 |
|---|---|---|---|---|---|
| Plasma creatinine (µmol/L) | 61.60±14.21 | 90.33±26.26 | 142.87±63.91 | 202.03±53.21 | 590.40±277.87 |
| Urine creatinine (µmol/L) | 12494±6926.21 | 10270.07±6238.45 | 7359.17±3882.42 | 6447.93±4354.35 | 4268.64±5782.24 |
| Glomerular filtration rate | 132.53±33.21 | 76.98±9.1 | 45.81±8.5 | 24.73±4.56 | 7.84±2.9 |
| Urine 8-oxo-dGsn/Cr (µmol/mol) | 1.87±0.87 | 1.92±1.01 | 1.84±0.81 | 1.47±0.89 | 1.60±1.59 |
| Urine 8-oxo-Gsn/Cr (µmol/mol) | 3.07±1.07 | 3.42±1.34 | 3.72±1.47 | 3.90±1.93 | 3.75±2.26 |
| Plasma 8-oxo-Gsn/Cr (µmol/mol) | 0.17±0.12 | 0.24±0.18 | 0.37±0.20 | 0.49±0.22 | 1.10±0.57 |
| Plasma/urine 8-oxo-Gsn | 0.02±0.02 | 0.03±0.02 | 0.06±0.04 | 0.10±0.05 | 0.34±0.03 |

Data were expressed as mean ± SD; significant differences were considered when P <0.05

## Claims

1. A method for detecting a disease in a mammal, the method comprising:
(1) detecting a marker in a sample of a subject, wherein the marker is a ratio of 8-oxo-guanosine (8-oxoGsn) concentration to creatinine concentration;
(2) comparing the marker with a reference, wherein a difference of the marker compared with the reference is used to evaluate the disease status of the subject;
wherein the disease is AIDS, hepatitis B, hepatitis C, syphilis, liver cancer, coronary heart disease, or acute coronary syndrome.

2. The method according to claim 1, wherein the sample is a body fluid.

3. The method according to claim 2, wherein the body fluid is selected from the group consisting of peripheral blood, serum, plasma, synovial fluid, aqueous humor, breast milk, semen, prostate fluid, sweat, tear, pleural effusion, ascites fluid, pericardial fluid, chyle, bile, interstitial fluid, menstrual blood, vomitus, vaginal secretion, mucosal secretion, pancreatic juice, bronchopulmonary suction fluid, blastocyst cavity fluid, umbilical cord blood, urine, cerebrospinal fluid, saliva, lymph fluid and excreta.

4. The method according to claim 3, wherein the body fluid is urine, peripheral blood, serum, or plasma.

5. The method according to claim 1, wherein the reference is any one or both of the following:
(1) an interval value of 8-oxo-guanosine (8-oxoGsn) level of a healthy mammal;
(2) an interval value of the ratio of 8-oxo-guanosine (8-oxoGsn) concentration to creatinine concentration of a healthy mammal.

6. The method according to claim 5, wherein an interval value is 95% percentile.

7. The method according to any one of claims 1 to 6, wherein the mammal is a human.

8. A use of a kit in a method of any one of claims 1 to 7, the kit comprising 8-oxo-guanosine (8-oxoGsn) standard, 10mM ammonium acetate, methanol, 1 to 2000 pg/µL isotope internal standard [¹³C,¹⁵N₂]8-oxo-deoxyguanosine (8-oxo-dGsn), and 1 to 2000 pg/µL isotope internal standard [¹⁵N₅]8-oxo-guanosine (8-oxoGsn).

9. The use of a kit according to claim 8, wherein the isotope internal standards have a concentration of 100 to 800 pg/µL.

10. The use of a kit according to claim 8, wherein the 10 mM ammonium acetate and methanol are 70% methanol + 30% 10 mM ammonium acetate solution, and the solution after mixing has a pH of 3.5 to 4.0.

## Patentansprüche

1. Verfahren zum Nachweis einer Krankheit bei einem Säugetier, wobei das Verfahren Folgendes umfasst:
(1) Nachweis eines Markers in einer Probe eines Probanden, wobei der Marker ein Verhältnis der 8-Oxo-Guanosin (8-oxoGsn)-Konzentration zur Kreatinin-Konzentration ist;
(2) Vergleichen des Markers mit einer Referenz, wobei eine Differenz des Markers im Vergleich zur Referenz verwendet wird, um den Krankheitsstatus des Probanden zu bewerten;
wobei die Krankheit AIDS, Hepatitis B, Hepatitis C, Syphilis, Leberkrebs, koronare Herzkrankheit oder akutes Koronarsyndrom ist.

2. Verfahren nach Anspruch 1, wobei die Probe eine Körperflüssigkeit ist.

3. Verfahren nach Anspruch 2, wobei die Körperflüssigkeit aus der Gruppe bestehend aus peripherem Blut, Serum, Plasma, Synovialflüssigkeit, Kammerwasser, Muttermilch, Sperma, Prostataflüssigkeit, Schweiß, Tränen, Pleuraerguss, Aszitesflüssigkeit, Perikardflüssigkeit, Chylus, Galle, interstitieller Flüssigkeit, Menstruationsblut, Erbrochenem, Vaginalsekret, Schleimhautsekret, Pankreassaft, bronchopulmonaler Absaugflüssigkeit, Blastozystenhöhlenflüssigkeit, Nabelschnurblut, Urin, Liquor cerebrospinalis, Speichel, Lymphflüssigkeit und Ausscheidungen ausgewählt ist.

4. Verfahren nach Anspruch 3, wobei die Körperflüssigkeit Urin, peripheres Blut, Serum oder Plasma ist.

5. Verfahren nach Anspruch 1, wobei die Referenz eine der folgenden oder beide ist:
(1) ein Intervallwert des 8-Oxo-Guanosin (8-OxoGsn)-Spiegels eines gesunden Säugetiers;
(2) ein Intervallwert des Verhältnisses der 8-Oxo-Guanosin (8-OxoGsn)-Konzentration zur Kreatinin-Konzentration eines gesunden Säugetiers.

6. Verfahren nach Anspruch 5, wobei ein Intervallwert 95% Perzentil ist.

7. Verfahren nach einem der Ansprüche 1 bis 6, wobei das Säugetier ein Mensch ist.

8. Verwendung eines Kits in einem Verfahren nach einem der Ansprüche 1 bis 7, wobei das Kit 8-Oxo-Guanosin (8-OxoGsn)-Standard, 10mM Ammoniumacetat, Methanol, 1 bis 2000 pg/µL isotopeninternen Standard [¹³C,¹⁵N₂]8-Oxo-Desoxyguanosin (8-Oxo-dGsn) und 1 bis 2000 pg/µL isotopeninternen Standard [¹⁵N₅]⁸-Oxo-Guanosin (8-OxoGsn) umfasst.

9. Verwendung eines Kits nach Anspruch 8, wobei die isotopeninternen Standards eine Konzentration von 100 bis 800 pg/µL aufweisen.

10. Verwendung eines Kits nach Anspruch 8, wobei das 10 mM Ammoniumacetat und Methanol eine Lösung aus 70% Methanol + 30% 10 mM Ammoniumacetat sind und die Lösung nach dem Mischen einen pH-Wert von 3,5 bis 4,0 aufweist.

## Revendications

1. Procédé de détection d'une maladie chez un mammifère, le procédé comprenant :
(1) détecter un marqueur dans un échantillon d'un sujet, dans lequel le marqueur est un rapport d'une concentration en 8-oxo-guanosine (8-oxoGsn) à une concentration en créatinine;
(2) comparer le marqueur avec une référence, dans lequel une différence du marqueur par rapport à la référence est utilisée pour évaluer l'état de la maladie chez le sujet;
dans lequel la maladie est le SIDA, l'hépatite du type B, l'hépatite du type C, la syphilis, le cancer du foie, la coronaropathie ou le syndrome coronarien aigu.

2. Procédé selon la revendication 1, dans lequel l'échantillon est un fluide corporel.

3. Procédé selon la revendication 2, dans lequel le fluide corporel est sélectionné dans le groupe consistant en le sang périphérique, le sérum, le plasma, le liquide synovial, l'humeur aqueuse, le lait maternel, le sperme, le liquide prostatique, la sueur, les larmes, l'épanchement pleural, le liquide d'ascite, le liquide péricardique, la chyle, la bile, le liquide interstitiel, le sang menstruel, le vomi, la sécrétion vaginale, la sécrétion muqueuse, le jus pancréatique, le liquide de succion bronchopulmonaire, le liquide de cavité blastocystique, le sang du cordon ombilical, l'urine, le liquide cérébrospinal, la salive, la lymphe et les excréments.

4. Procédé selon la revendication 3, dans lequel le fluide corporel est l'urine, le sang périphérique, le sérum ou le plasma.

5. Procédé selon la revendication 1, dans lequel la référence est l'un ou deux des suivants:
(1) une valeur d'intervalle d'un niveau de 8-oxo-guanosine (8-oxoGsn) d'un mammifère sain;
(2) une valeur d'intervalle du rapport d'une concentration en 8-oxo-guanosine (8-oxoGsn) à une concentration en créatinine d'un mammifère sain.

6. Procédé selon la revendication 5, dans lequel une valeur d'intervalle est de 95e percentile.

7. Procédé selon l'une quelconque des revendications 1 à 6, dans lequel le mammifère est un être humain.

8. Utilisation d'un kit dans un procédé selon l'une quelconque des revendications 1 à 7, le kit comprenant un standard de 8-oxo-guanosine (8-oxoGsn), l'acétate d'ammonium 10 mM, le méthanol, un standard interne isotopique [¹³C,¹⁵N₂]8-oxo-désoxyguanosine (8-oxo-dGsn) à 1 à 2000 pg/µL, et un standard interne isotopique [¹⁵N₅]8-oxo-guanosine (8-oxoGsn) à 1 à 2000 pg/µL.

9. Utilisation d'un kit selon la revendication 8, dans laquelle les standards internes isotopiques présentent une concentration de 100 à 800 pg/µL.

10. Utilisation d'un kit selon la revendication 8, dans laquelle l'acétate d'ammonium 10 mM et le méthanol sont sous forme d'une solution de 70 % de méthanol + 30 % d'acétate d'ammonium 10 mM, et la solution après mélange présente un pH de 3,5 à 4,0.
